# EUROPEAN PATENT APPLICATION

(11) **EP 4 382 176 A1**
(43) Date of publication of application: **12.06.2024**
(21) Application number: 22853018.4
(22) Date of filing: 01.08.2022
(51) Int. Cl.: A61Q 19/00, A61K 8/73

(54) **SEBUM SECRETION INHIBITOR**

(30) Priority: 02.08.2021 JP 2021126887
(71) Applicant: Soken Co., Ltd., Kagawa 769-0210 (JP)
(72) Inventor: TOKUYAMA, Takashi, Ayauta-gun, Kagawa 769-0210 (JP); TOKUYAMA, Takahito, Ayauta-gun, Kagawa 769-0210 (JP); AYAKI, Satomi, Ayauta-gun, Kagawa 769-0210 (JP)
(74) Representative: Hoffmann Eitle
(86) International application number: PCT/JP2022/029533
(87) International publication number: WO 2023/013599

(57) **Abstract**

An object is to provide a novel sebum secretion inhibition. Provided is a sebum secretion suppressor which is an agent containing a polysaccharide, in which at least a part of constituent sugars constituting the polysaccharide is one or more {for example, arabinogalactan, lipopolysaccharide (LPS), arabinoxylan and/or glucomannan} selected from the group consisting of arabinose, galactose, and xylose.

## Description

### Technical Field

The present invention relates to a sebum secretion suppressor.

### Background Art

Sebum is a semifluid oil-and-fat substance secreted from sebaceous glands. Sebum serves to moisturize skin or hair and prevent dryness. On the other hand, excessive secretion of sebum in the skin causes acne, oily skin, stickiness, and the like, and thus tends to be unattractive for cosmetic purposes. Therefore, there is a demand for a medicine or a cosmetic that appropriately inhibits the secretion of sebum in the skin.

Under this demand, for example, Patent Literature 1 to 3 propose various components having a sebum secretion inhibiting effect.

### Citation List

### Patent Literature

Patent Literature 1: JP 2013-028572 A
Patent Literature 2: JP 2016-150916 A
Patent Literature 3: JP 2014-237604 A

### Summary of Invention

### Technical Problem

An object of the present invention is to provide novel sebum secretion suppressor.

### Solution to Problem

The present invention (1) is a sebum secretion suppressor which is an agent containing a polysaccharide, in which at least some of constituent sugars constituting the polysaccharide are one or more selected from the group consisting of arabinose, galactose, and xylose.

The present invention (2) is the sebum secretion suppressor of the invention (1), in which the polysaccharide is water-soluble.

The present invention (3) is the sebum secretion suppressor of the invention (1) or (2), in which a molecular weight of the polysaccharide is 5000 or more.

The present invention (4) is the sebum secretion suppressor according to any one of the inventions (1) to (3), in which the polysaccharide is galactan, arabinogalactan, galacturonan, lipopolysaccharide (LPS), arabinoxylan and/or glucomannan.

The present invention (5) is the sebum secretion suppressor according to any one of the inventions (1) to (4), further including a rice-derived component.

The present invention (6) is the sebum secretion suppressor according to the invention (5), in which the rice-derived component is one or more of (1) a pulverized product of rice, (2) an extract of rice, (3) an extract obtained by adding water or an organic solvent to rice, treating rice with an acid or an alkali, causing an enzyme or koji to act on a hydrate of rice, or performing these treatments under heating or not heating, (4) a product obtained by causing an enzyme or koji to act on rice before, at the same time as, or after the extraction when extracting rice, and (5) a product obtained by performing alcohol fermentation or organic acid fermentation on an extract of rice or an extract obtained by causing an enzyme or koji to act.

### Brief Description of Drawings

Fig. 1 is a diagram showing a degree of a sebum secretion inhibiting effect of agents according to Examples 1 to 7 and Comparative Examples 1 and 2.
Fig. 2 is a diagram showing the sebum secretion inhibiting effect of agents according to Examples 1 to 7 and Comparative Examples 1 and 2 (stain photograph).

### Description of Embodiments

In a sebum secretion suppressor according to the present invention which is an agent containing a polysaccharide, at least a part of constituent sugars constituting the polysaccharide is one or more selected from the group consisting of arabinose, galactose, and xylose. Hereinafter, the sebum secretion suppressor will be described in detail.

### <<Component>>

### <Polysaccharide>

### (Constituent Sugars)

In the polysaccharide contained in the sebum secretion suppressor, at least a part of constituent sugars constituting the polysaccharide is one or more selected from the group consisting of arabinose, galactose, and xylose.

Here, arabinose, galactose, and xylose may be D-type, L-type, α-type, or β-type.

In addition, arabinose preferably has a glycosidic bond in the form of, for example, a β1,6 bond and an α1,3 bond in the polysaccharide. Galactose preferably has a glycosidic bond in the form of, for example, a β1,4 bond, a β1,3 bond, a β1,6 bond, and an α1,4 bond in the polysaccharide. Furthermore, xylose preferably has a glycosidic bond in the form of, for example, a β1,4 bond and an α1,3 bond in the polysaccharide. In addition, the constituent sugar (arabinose, galactose, and xylose) may be present in either or both of the main chain and a branched side chain of the polysaccharide.

Next, in a case where arabinose is an essential constituent sugar, the amount of arabinose is preferably 0.1% to 90%, more preferably 1% to 80%, and still more preferably 2% to 70%, based on the total amount (mass) of constituent sugars constituting the polysaccharide. In addition, in a case where galactose is an essential constituent sugar, the amount of galactose is preferably 0.1% to 100%, more preferably 1% to 90%, and still more preferably 2% to 80%, based on the total amount of constituent sugars constituting the polysaccharide. Furthermore, in a case where xylose is an essential constituent sugar, the amount of xylose is preferably 0.1% to 90%, more preferably 1% to 80%, and still more preferably 2% to 70%, based on the total amount of constituent sugars constituting the polysaccharide.

In a case where a constituent sugar constituting the polysaccharide is present in addition to one or more constituent sugars selected from the group consisting of arabinose, galactose, and xylose, the constituent sugar is not particularly limited, and may be a monosaccharide (for example, aldoses such as glyceraldehyde, erythrose, threose, ribose, lyxose, xylose, allose, talose, gulose, glucose, altrose, mannose, and idose; ketoses such as dihydroxyacetone, erythrulose, ribulose, psicose, fructose, sorbose, and tagatose), a disaccharide (for example, sucrose, lactulose, lactose, maltose, trehalose, cellobiose, and the like), and/or a polysaccharide (for example, glycogen, cellulose, chitin, agarose, carrageenan, heparin, pectin, xyloglucan, and the like).

### (Preferable Polysaccharide)

The polysaccharide is not particularly limited, and may be a glycoprotein, a proteoglycan, a glycosaminoglycan, a glycolipid, or the like. Also, the polysaccharide may be modified (protein modification, phosphate group, sulfate group, lipid binding, acetylation, amination, methylation, and other chemical modifications). Here, suitable polysaccharides are galactan, arabinogalactan, galacturonan, lipopolysaccharide (LPS), arabinoxylan and/or glucomannan. Note that these polysaccharides are available as commercial products, and for example, β-1,4-galactan is available from Megazyme; Type II arabinogalactan is available from Tokyo Chemical Industry Co., Ltd., sigma, Megazyme, and Lonza; Rhamnogalacturonan I is available from Megazyme; LPS is available from sigma, Fujifilm Wako Pure Chemical Industries, Ltd., and Nacalai Tesque Inc.; arabinoxylan is available from Megazyme, Furmatech Co., Ltd., and CBC Co., Ltd.; glucomannan is available from Shimizu Chemical Co., Ltd., Magazyme, and Harada Food Co., Ltd.

In addition, the molecular weight of the polysaccharide is preferably 5000 or more, more preferably 10000 or more, and still more preferably 50000 or more. Within this range, superior effects can be expected. Note that an upper limit value is not particularly limited, and is, for example, 2 million. Here, the molecular weight is a peak top average molecular weight (Mp). Here, a method for measuring the peak top average molecular weight is as follows. Note that a column (1) and a column (2) are selectively used depending on a target molecular weight size {in a case where the target molecular weight sizes overlap, the value in the column (1) is prioritized}.

### [Analysis Method]

Measurement apparatus:
   · High performance liquid chromatography apparatus: HPLC
Conditions:
   · Mobile phase: pure water, 100%
   · Detector: ELSD (evaporative light scattering detector)
   · Flow rate: 0.5 mL/min
   · Column temperature: 30°C
   · Column (1): OHpak LB-805 (manufactured by Shodex)/target molecular weight range 100,000 to 1 million/exclusion limit molecular weight 4 million
   · Column (2): OHpak LB-804 (manufactured by Shodex)/target molecular weight range 5,000 to 400,000/exclusion limit molecular weight 1 million
   · Standard molecular weight: Pullulan standard set (manufactured by Shodex)
Calculation:
   · Prepare calibration curve of molecular weight and peak top detection time with standard molecular weight, and calculate average molecular weight from detection time of peak top of sample

Here, the polysaccharide may be water-soluble or poorly soluble, and may also be nonionic or ionic (cationic or anionic). However, it is preferably water-soluble. When it is water-soluble, it is preferable in that it easily dissolves in a water-based external preparation for skin such as a skin lotion, it readily takes a form that is comfortable for use for humans or animals with oily skin in which sebum secretion is desired to be inhibited, and it is easy to formulate formulations therewith for subcutaneous injection or the like. Here, the term "water-soluble" means that 1% by mass or more of the polysaccharide dissolves in water at 25°C.

### <Other Components>

The sebum secretion suppressor according to the present invention preferably further includes, in addition to the polysaccharides described above, one or more rice-derived components of (1) a pulverized product of rice, (2) an extract of rice, (3) an extract obtained by adding water or an organic solvent to rice, treating rice with an acid or an alkali, causing an enzyme or koji to act on a hydrate of rice, or performing these treatments under heating or not heating, (4) a product obtained by causing an enzyme or koji to act on rice before, at the same time as, or after the extraction when extracting rice, and (5) a product obtained by performing alcohol fermentation or organic acid fermentation on an extract of rice or an extract obtained by causing an enzyme or koji to act. Hereinafter, the rice-derived components will be described.

First, "rice" is a concept including not only rice such as white rice, brown rice, and germinated rice but also a part of rice such as white bran and red bran. However, it is preferable to use rice essentially containing a white rice portion, that is, white rice, brown rice, germinated rice, and white bran. Here, the white rice and the brown rice refer to brown rice and white rice such as non-glutinous rice and glutinous rice regardless of whether Japonica rice and Indica rice, and a variety or a type does not matter. In addition, white bran and red bran generally refer to 92% or more of red bran or 92% or less of white bran that appear at the time of milling, but a mixture of both may be used. Since an active ingredient is stable to heat and light, the above raw materials may be subjected to raw material treatment such as surface modification such as immersion, steaming, roasting (refers to all of sand roasting, net roasting, hot air roasting, and the like), steaming roasting, and freeze drying, light modification such as UV irradiation, pressure roasting such as pot rice, and frying. White rice, brown rice, and germinated rice are effective even if they are used as they are, but it is preferable that they are used after being pulverized in terms of practical use. White rice, brown rice, and germinated rice may be pulverized into a powdery material by a general method using a pulverizer or a rice mill.

In a case where the germinated rice is produced, rice with germ is immersed in water or sprayed with water to germinate. A temperature at the time of germination is 10°C to 50°C. However, the temperature and time are not limited as long as germination occurs. In addition, in a case where there is a risk that the water spoils during germination, it is preferable to replace the water so as not to spoil or to perform some preservation. Here, germination refers to all from immediately before germination to germination. The germinated rice is thoroughly washed and used. In this case, it may be dried and used. In a case of extracting or enzymatically decomposing rice or causing koji to act thereon, pulverizing the rice as a raw material into granules or powdery materials increases the surface area and thus improves the efficiency. It is not necessary to perform pulverizing, but in this case, it takes a long time to decompose and extract a rice tissue.

In a case of extracting rice with water, a high extraction temperature is efficient, but extraction can be sufficiently performed even at a low temperature. However, in a case of a low temperature of 40°C or lower, it is desirable to make a pH acidic or alkaline, or add a preservative or alcohol to treat the rice so as not to spoil. The extraction time may be long or short as long as the active ingredient can be extracted, and may be determined according to the extraction temperature. In addition, the extraction may be performed under pressure, normal pressure, or reduced pressure. In a case of extraction with water, the most problematic issue is the gelatinization phenomenon. When becoming pasty, not only does the extraction efficiency deteriorate, but also the actual work is extremely difficult. In order to prevent this, starch may be broken down by reaction with addition of amylase or by setting to acidic with hydrochloric acid or the like, and by using this method, this problem can be sufficiently solved, and with no problem in practical use at all.

Since the active ingredient in the extract is stable to acid and alkali, it is also effective to perform acid decomposition extraction or alkali decomposition extraction. In this case, neutralization and desalination are performed as necessary. Even in a case of extraction with an organic solvent, it is desirable that the rice is extracted by being finely pulverized or pulverized into a powdery material as much as possible. The organic solvent may be a general organic solvent such as an alcohol, acetone, n-hexane, ethyl acetate, or methanol, but a safe organic solvent may be used because it is necessary to completely remove the solvent after extraction when the organic solvent is harmful to the human body. In addition, rice may be enzymatically decomposed or koji may be caused to act thereon. The enzymatic decomposition as used herein refers to causing one enzyme or two or more enzymes acting on rice, such as a starch degrading enzyme (liquefying enzyme or diastatic enzyme), a proteolytic enzyme, a lipolytic enzyme, a fibrolytic enzyme, a lignin degrading enzyme, and a pectin degrading enzyme to act. Examples thereof can include a combination of the liquefying enzyme and the diastatic enzyme. In addition, a type of koji mold as the koji and the variety and type of rice are not limited. In addition, acid or alkaline extraction, organic solvent extraction, enzymatic decomposition, and koji action may be performed in combination.

In addition, when performing the extraction, the enzymatic decomposition or causing the koji to act may be performed before the extraction, simultaneously with the extraction, or after the extraction.

Furthermore, when performing the treatment, organic acid fermentation such as alcohol fermentation, lactic acid fermentation, or acetic acid fermentation may be performed at the same time as or after the above treatment. In addition, in a case where alcohol fermentation is performed, concentration is easily performed and it becomes easy to concentrate the active ingredient. Furthermore, the saccharide may be removed by performing aeration fermentation with yeast, alcohol precipitation, or the like. The fermentation form may be any of a moromi fermentation and a liquid fermentation, but the moromi fermentation is preferable. These fermentations may be repeated twice or more, or different fermentation methods may be combined.

The rice-derived component obtained as described above is used as it is, or after being squeezed or filtered, without separating a residue. When used as it is, sterilization or antibacterializing is performed to obtain a product. In a more preferred embodiment, it is obtained by heating (for example, boiling) water to which rice bran and various enzymes (preferably, one or more selected from the group consisting of a proteolytic enzyme, a lipolytic enzyme, a fibrolytic enzyme, an amylolytic enzyme, and a pectinolytic enzyme; and particularly preferably, a combination of an amylolytic enzyme, a proteolytic enzyme, a lipolytic enzyme, and a fibrolytic enzyme) are added and then subjecting to fermentation (for example, alcohol fermentation and/or lactic acid fermentation; and preferably, alcohol fermentation after lactic acid fermentation).

In addition, the sebum secretion suppressor according to the present invention may contain various pharmaceutically acceptable carriers. Examples thereof can include components usually added to pharmaceuticals, quasi-pharmaceutical products, and cosmetic products (for example, a skin cosmetic, a bathing agent, or a cleaning agent), specifically, excipients, binders, disintegrants, lubricants, flavoring agents, coloring agents, solubilizing agents, suspending agents, emulsifiers, coating agents, moisturizing agents, cleaning agents, ultraviolet absorbers, and drugs. Specifically, for example, in a case of use as a cosmetic, an aqueous component, an oily component, a powder, a surfactant, an oil agent, a pH adjusting agent, a preservative, an alcohol, an antioxidant, a thickener, a pigment, a perfume, an animal extract, a plant extract, and the like, which are commonly used in cosmetics, are appropriately formulated as necessary to be adjusted. In addition, the sebum secretion suppressor according to the present invention may be used in combination with other sebum secretion suppressors.

### «Application»

### (Application Subject)

A subject to which the sebum secretion suppressor according to the present invention is applied is not particularly limited, and may be a human (for example, a healthy person, a person with greasy skin, and a patient with seborrheic dermatitis) or an animal other than a human (for example, a dog or a cat).

### (Application Method)

The sebum secretion suppressor according to the present invention is applied to the skin (for example, coating, spraying, and the like) and used (typically, a transdermal external preparation). For example, it can be used as a pharmaceutical product such as an external preparation for skin, a cleaning agent, and a bathing agent, a quasi-pharmaceutical product, and a cosmetic product. Specifically, it can be provided in various dosage forms such as a solubilizing system (liquid skin external preparation, skin lotion, and the like) such as a lotion, an emulsifying system (emulsion foundation, O/W emulsion type serum, and the like) such as an emulsion, a powder-granule system (bath cosmetic and the like), a cream system (O/W emulsion type cream, hand cream, makeup base cream, and the like), an ointment system (O/W type emulsifiable ointment and the like), and a gas system (aerosol and the like). More specifically, in addition to the above-described transdermal external preparation, subcutaneous injection, a microneedle patch, a cataplasm, and a sheet mask can also be mentioned.

### (Application Amount)

In a case where the sebum secretion suppressor according to the present invention is applied to the skin, it is preferable to use a sebum secretion suppressor containing 0.0001 mass% or more of the polysaccharide (polysaccharide containing one or more constituent sugars selected from the group consisting of arabinose, galactose, and xylose) based on the total mass of the sebum secretion suppressor to be applied, and it is more preferable to use a sebum secretion suppressor containing 0.001 mass% or more of the polysaccharide. An upper limit value is not particularly limited, and is, for example, 100 mass%.

### «Examples»

Hereinafter, the present invention will be described more specifically with reference to Examples, but the present invention is not limited to the Examples.

### <Preparation of Sebum Secretion Suppressor>

### (Example 1)

A β-1,4-galactan aqueous solution was added to pure water to have a concentration of 1 mg/mL to obtain an agent according to Example 1. As β-1,4-galactan, one having a molecular weight of 1182 kDa, galactose of 82.0%, arabinose of 5.8%, rhamnose of 5.1%, and xylose of 1.4% was used.

### (Example 2)

An aqueous solution of type II arabinogalactan was added to pure water to have a concentration of 100 mg/mL to obtain an agent according to Example 2. As the type II arabinogalactan, one commercially available from Tokyo Chemical Industry Co., Ltd. was used.

### (Example 3)

An aqueous solution of rhamnogalacturonan I was added to 10% EtOH and 0.005 N NaOH aqueous solution to have a concentration of 25 mg/mL to obtain an agent according to Example 3. As rhamnogalacturonan I, one containing galactose of 23.1%, arabinose of 1.0%, and xylose of 0.8% was used.

### (Example 4)

An arabinoxylan aqueous solution was added to 10% EtOH and 0.005 N NaOH aqueous solution to have a concentration of 25 mg/mL to obtain an agent according to Example 4. As arabinoxylan, one having a molecular weight of 56.7 kDa, arabinose of 38%, and xylose of 62% was used.

### (Example 5)

A glucomannan aqueous solution was added to pure water to have a concentration of 5 mg/mL to obtain an agent according to Example 5. As the glucomannan, glucomannan manufactured by Shimizu Chemical Co., Ltd. was used.

### (Example 6)

The LPS aqueous solution was added to pure water to satisfy 5 µg/mL to obtain an agent according to Example 6. As the LPS, LPS manufactured by sigma was used.

### (Comparative Example 1)

An aqueous solution of β-1,3-glucan was added to a Sebocell differentiation medium containing 0.002% EtOH and 0.01 M NaCl to have a concentration of 4 mg/mL to obtain an agent according to Comparative Example 1. In a case where the raw material was dissolved in an aqueous solution, since the raw material was gelatinized and difficult to handle, the raw material was directly dissolved in a medium.

### (Comparative Example 2)

2 g of an amylolytic enzyme, 2 g of a proteolytic enzyme, 2 g of a lipolytic enzyme, 2 g of a fibrolytic enzyme, and 1500 ml of water were added to 500 g of white rice, and allowed to stand at 50°C for 20 hours. Thereafter, a temperature was gradually increased, and the mixture was extracted by boiling for 5 minutes and then filtered to obtain 1.2 L of an agent (rice extract) according to Comparative Example 2.

### (Example 7)

A β-1,4-galactan aqueous solution was added to the rice extract obtained in Comparative Example 2 to have a concentration of 1 mg/mL to obtain an agent according to Example 7.

### <Test Method>

### (Cell Culture)

Hamster derived sebaceous gland cells were seeded at a cell density of 7.5 × 10⁴ cells/well in a 24 well plate for tissue cells, and cultured in a Sebocell growth medium (10% FBS, 4 mM Glutamine, 10 ng/mL EGF-containing DMEM/F12-HAM) at 37°C·5% CO₂ concentration until confluent. Thereafter, the medium was replaced with a Sebocell differentiation medium (10% FBS, 4 mM Glutamine, 10 µg/mL Insulincontaining DMEM/F12-HAM) to which the agent according to Examples 1 to 8 and Comparative Example 2 was added, and Comparative Example 1. The medium was replaced with the same Sebocell differentiation medium every other day, and the cells were cultured for one week.

### <WST-1 Measurement>

After completion of the culture, 4-[3-(iodophenyl)-2-(4-nitrophenyl)-2H-5-tetrazolino]-1,3-benzenedisulfonate (tetrazolium salt WST-1) as a reagent for cell proliferation measurement was added to each well, and cultured at 37°C·5% CO₂ concentration for 20 minutes, then a culture solution was gently stirred, and then recovered. An absorbance at 450 nm of the resulting culture solution was measured, and a value obtained by subtracting the absorbance at 450 nm of a background control (culture medium without cells + tetrazolium salt WST-1) was used as an index of the number of living cells.

### <Oil Red O Staining>

After measurement of living cells, the cells were washed twice with PBS, and a formalin solution prepared to have a 10% concentration (v/v) with PBS was added. After the cells were fixed by allowing the cells to stand at room temperature for 10 minutes, the supernatant was removed and the cells were washed twice with PBS. An Oil Red O stock solution prepared to have a concentration (w/v) of 0.3% with 100% isopropanol was further diluted with pure water so as to have a concentration (v/v) of 60%, and filtered (0.2 mm) to prepare an Oil Red O stain solution. This was added to the cells blended with 60% isopropanol for 1 minute, and the cells were allowed to stand at room temperature for 30 minutes for staining. Thereafter, the cells were washed twice with 60% isopropanol to remove excess dye, and replaced with PBS to take a photograph of microscopic observation images of the cells. Thereafter, the supernatant was removed, 100% isopropanol was added, and the mixture was shaken at room temperature for 5 minutes. The amount of lipid synthesis was examined by measuring the absorbance at 530 nm of the extracted dye. The lipid amount per cell number was calculated by dividing the obtained value by the index of each living cell number measured by WST-1. Relative values were calculated for the amount of lipid in each test sample when the amount of lipid to which no test sample was added was 1.0.

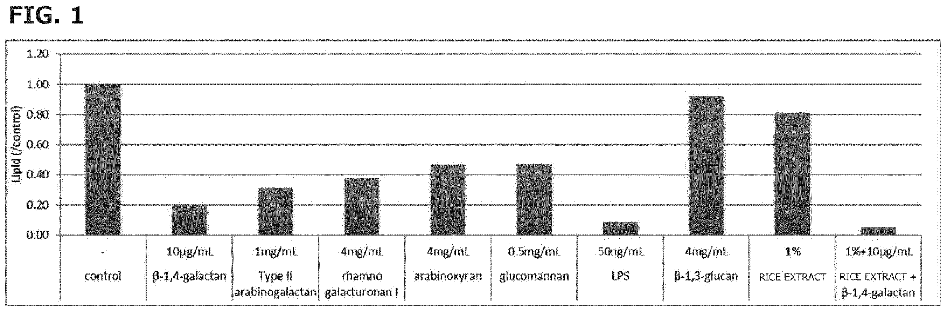

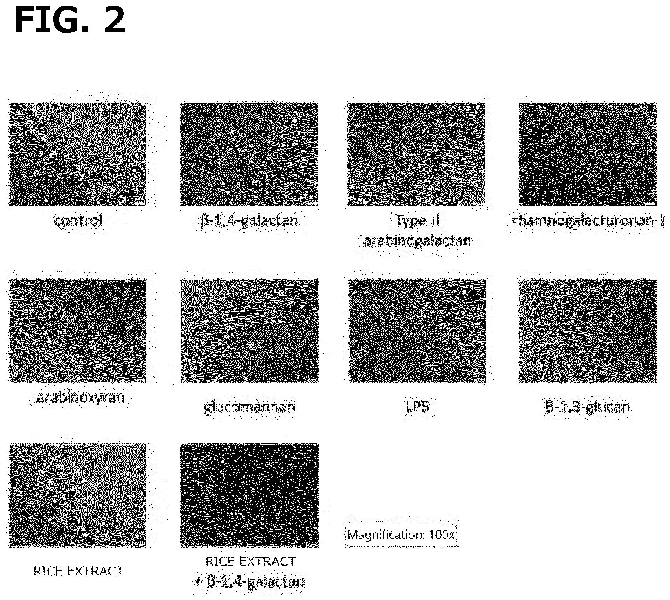

The results are shown in Figs. 1 and 2. The concentration in the drawing is the concentration of each component in the medium.

## Claims

1. A sebum secretion suppressor comprising a polysaccharide composed of arabinogalactan, lipopolysaccharide (LPS), arabinoxylan and/or glucomannan.

2. The sebum secretion suppressor according to claim 1, wherein the polysaccharide is water-soluble.

3. The sebum secretion suppressor according to claim 1, wherein a molecular weight of the polysaccharide is 5000 or more.

4. The sebum secretion suppressor according to any one of claims 1 to 3, further comprising a rice-derived component.

5. The sebum secretion suppressor according to claim 4, wherein the rice-derived component is one or more of (1) a pulverized product of rice, (2) an extract of rice, (3) an extract obtained by adding water or an organic solvent to rice, treating rice with an acid or an alkali, causing an enzyme or koji to act on a hydrate of rice, or performing these treatments under heating or not heating, (4) a product obtained by causing an enzyme or koji to act on rice before, at the same time as, or after the extraction when extracting rice, and (5) a product obtained by performing alcohol fermentation or organic acid fermentation on an extract of rice or an extract obtained by causing an enzyme or koji to act.
